(19) Europäisches Patentamt
European Patent Office
Office européen des brevets

(11) Veröffentlichungsnummer: **0 341 478 A2**

(12) **EUROPÄISCHE PATENTANMELDUNG**

(21) Anmeldenummer: **89107347.0**

(51) Int. Cl.[4]: **C07D 213/61**

(22) Anmeldetag: **24.04.89**

(30) Priorität: **07.05.88 DE 3815727**
**11.11.88 DE 3838243**

(43) Veröffentlichungstag der Anmeldung:
**15.11.89 Patentblatt 89/46**

(84) Benannte Vertragsstaaten:
**BE CH DE ES FR GB IT LI NL**

(71) Anmelder: **BAYER AG**

**D-5090 Leverkusen 1 Bayerwerk(DE)**

(72) Erfinder: **Lindel, Hans, Dr.**
**Carl-Duisberg-Strasse 321**
**D-5090 Leverkusen 1(DE)**
Erfinder: **Hallenbach, Werner, Dr.**
**Kleiststrasse 10**
**D-4018 Langenfeld(DE)**
Erfinder: **Gallenkamp, Bernd, Dr.**
**Pau-Ehrlich-Strasse 13**
**D-5600 Wuppertal 1(DE)**

(54) **Verfahren zur Herstellung von 2,3-Dichlor-5-acetylpyridin.**

(57) Die vorliegende Erfindung betrifft Verfahren zur Herstellung von 2,3-Dichlor-5-acetylpyridin der Formel (I)

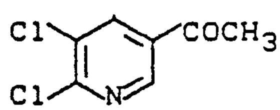

(I)

a) dadurch gekennzeichnet, daß man 5,6-Dichlornikotinsäurehalogenid der Formel (II)

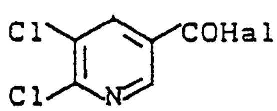

(II)

in welcher
Hal für Halogen steht,
mit Malonsäureestern der Formel (III)
$CH_2(COOR^1)_2$ (III)
in welcher
$R^1$ für $C_{1-3}$-Alkyl steht,
in Gegenwart von Magnesiumverbindungen und Basen umsetzt, anschließend schonend in Gegenwart wäßriger Säuren decarboxyliert, oder
b) dadurch gekennzeichnet, daß man 5,6-Dichlornikotinsäurehalogenid der Formel (II)

EP 0 341 478 A2

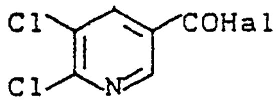

(II)

mit Methylmagnesiumhalogenid der Formel (V)
$H_3CMgHal$ (V)
in welcher
Hal für Halogen steht,
in Gegenwart von Katalysatoren umsetzt, oder

c) dadurch gekennzeichnet, daß man 5,6-Dichlornikotinsäure der Formel (VI)

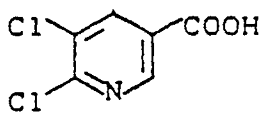

mit Methyllithium umsetzt,
sowie Verbindungen zur Durchführung dieser Verfahren und deren Herstellung.

## Verfahren zur Herstellung von 2,3-Dichlor-5-acetylpyridin

Die vorliegende Erfindung betrifft neue Verfahren zur Herstellung von 2,3-Dichlor-5-acetylpyridin, Zwischenprodukte zur Durchführung dieser Verfahren und deren Herstellung.

Pyridylalkylketone und ihre Verwendung als Zwischenprodukte zur Herstellung von Pyridylethanolaminen sind in einer nicht vorveröffentlichten Anmeldung der Anmelderin (Deutsche Patentanmeldung P 3 615 293.5) beschrieben.

Nach dem dort beschriebenen Verfahren werden sie erhalten, indem man Nikotinsäurealkylester mit Essigsäurealkylester in einer Claisen'schen Esterkondensation umsetzt und den so erhaltenen Pyridoylessigester verseift und decarboxyliert.

Die Pyridylethanolamine sind Wirkstoffe zur Förderung der Leistung von Tieren.

Gegenstand der vorliegenden Erfindung sind:

1. Verfahren zur Herstellung von 2,3-Dichlor-5-acetylpyridin der Formel (I)

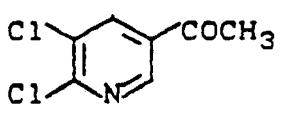

(I)

a) dadurch gekennzeichnet, daß man 5,6-Dichlornikotinsäurehalogenide der Formel (II)

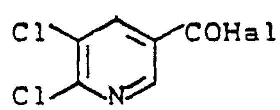

(II)

in welcher
Hal für Halogen steht,
mit Malonsäureestern der Formel (III)
$CH_2(COOR^1)_2$ (III)
in welcher
$R^1$ für $C_{1-3}$-Alkyl steht,
in Gegenwart von Magnesiumverbindungen und Basen umsetzt und anschließend schonend in Gegenwart wäßriger Säuren decarboxyliert, oder

b) dadurch gekennzeichnet, daß man Verbindungen der Formel (IV)

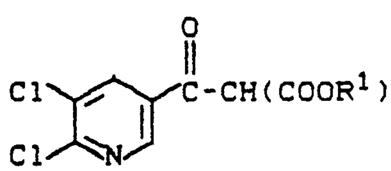

(IV)

in welcher
$R^1$ für $C_{1-3}$-Alkyl steht
in Gegenwart von Wasser, gegebenenfalls in Gegenwart eines organischen Lösungsmittels und gegebenenfalls in Gegenwart einer Protonensäure, bei Temperaturen zwischen 50 °C und 180 °C decarboxyliert, oder

c) dadurch gekennzeichnet, daß man 5,6-Dichlornikotinsäurehalogenide der Formel (II)

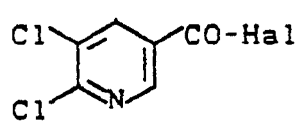

(II)

mit Methylmagnesiumhalogenid der Formel (V)
$H_3C\ MgHal$ (V)

in welcher
Hal für Halogen steht,
in Gegenwart von Katalysatoren umsetzt, oder

d) dadurch gekennzeichnet, daß man 5,6-Dichlornikotinsäure der Formel (VI)

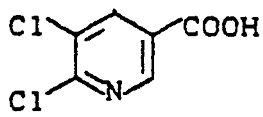

(VI)

mit Methyllithium umsetzt.

2. Verbindungen der Formel (IV)

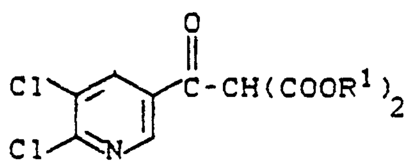

(IV)

in welcher
R' für $C_{1-3}$-Alkyl steht
sind neu.

3. Verfahren zur Herstellung der Verbindungen der Formel (IV) gemäß 2 (oben), dadurch gekennzeichnet, daß man Verbindungen der Formel (II)

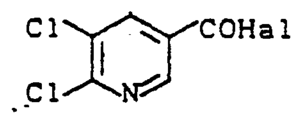

(II)

in welcher
Hal für Halogen steht,
mit Malonsäureestern der Formel (III)
$CH_2(COOR^1)_2$ (III)
in welcher
R' für $C_{1-3}$-Alkyl steht,
in Gegenwart von Magnesiumsalzen und Basen umsetzt.

Setzt man bei Verfahren 1a) als Verbindung der Formel (II) 5,6-Dichlornikotinsäurechlorid und als Verbindung der Formel (III) Malonsäuredimethylester ein, läßt sich das Verfahren durch das folgende Reaktionsschema darstellen:

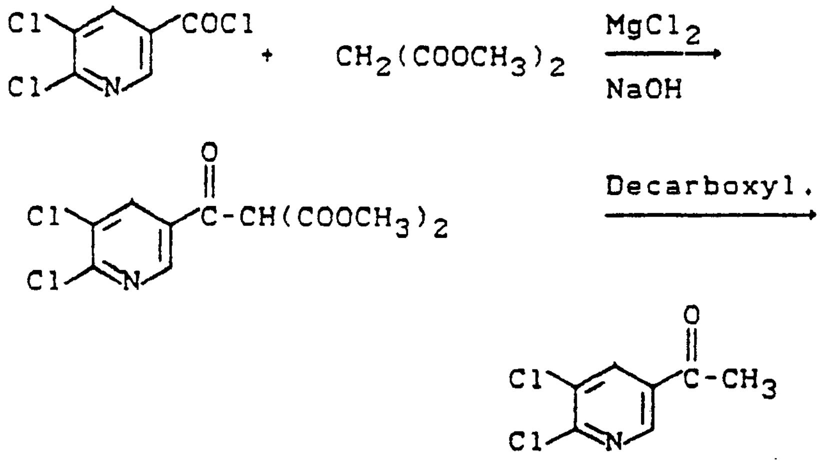


Verbindungen der Formel (II) sind bekannt (H. Meyer et al. Ber. Dt. Chem. Ges. 61 (1928), 2202). Im einzelnen seien folgende Verbindungen der Formel (II) genannt:
5,6-Dichlornikotinsäurechlorid, 5,6-Dichlornikotinsäurebromid.

Verbindungen der Formel (III) sind bekannt. Im einzelnen seien genannt:
Malonsäuredimethylester, -diethylester, -di-t-butylester, Meldrumsäure.

Die Reaktion wird durchgeführt, indem man die Nikotinsäurehalogenide mit Malonsäureester und Magnesiumsalz in Gegenwart von Basen und Verdünnungsmitteln umsetzt.

Die Umsetzung der Nikotinsäurehalogenide kann jedoch auch mit den entsprechenden Alkoxymagnesiummalonsäureestern erfolgen. Als solche seien genannt:
Methoxymagnesiummalonsäuredimethylester, Ethoxymagnesiummalonsäurediethylester. Diese Verbindungen sind z.B. bekannt aus Org. Synth. Col. Vol. IV (1963) 285.

Als Magnesiumsalze seien genannt:
Magnesiumchlorid, -bromid, -hydroxid, -oxid, -nitrat.

Als Basen seien genannt:
Alkali- und Erdalkalihydroxide, wie NaOH oder KOH, tertiäre Amine wie Triethylamin, Triethylendiamin, Trimethylen-tetrahydropyrimidin, Alkali- und Erdalkalialkoholate wie Na-Methylat, K-t-Butylat.

Als Verdünnungsmittel kommen praktisch alle inerten organischen Lösungsmittel in Frage. Hierzu gehören insbesondere aliphatische und aromatische, gegebenen falls halogenierte Kohlenwasserstoffe wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, außerdem Ester wie Essigsäuremethylester und -ethylester, ferner Nitrile wie z.B. Acetonitril und Propionitril, Benzonitril, Glutarsäuredinitril, darüber hinaus Amide wie z.B. Dimethylformamid, Dimethylacetamid und N-Methylpyrrolidon sowie Tetramethylensulfon, Dimethylsulfoxid und Hexamethylphosphorsäuretriamid.

Pro Mol des Nikotinsäurehalogenids der Formel (II) werden 1 - 3 Mol Malonsäureester der Formel (III) eingesetzt. Pro Mol Malonsäureester der Formel (III) werden 1 - 2, bevorzugt 1 - 1,5 Mol Magnesiumsalz und 2 - 4, bevorzugt 2 - 3 Mol Base eingesetzt.

Es wird bei Temperaturen von -20 °C bis 150 °C bevorzugt von -10 °C bis +60 °C gearbeitet.

Nach beendeter Reaktion wird das Gemisch mit Wasser verdünnt, angesäuert und der entstandene Nikotinoylmalonsäureester extrahiert.

Der Nikotinoylmalonsäureester der Formel (IV) wird in Gegenwart von Wasser und gegebenenfalls in Gegenwart von Protonensäuren bei Temperaturen zwischen 50 °C und 180 °C decarboxyliert.

Setzt man bei Verfahren 1b als Verbindung der Formel (IV) 5,6-Dichlornikotinoyl-malon-säuredimethylester ein, läßt sich das Verfahren durch das folgende Reaktionsschema darstellen:

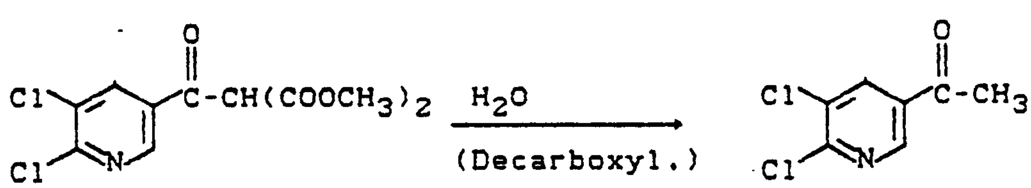

Die Reaktion wird durchgeführt, indem man den Nikotinoylmalonsäureester in Gegenwart von Wasser erhitzt.

Als Protonensäuren seien genannt:
Salzsäure, Schwefelsäure, Phosphorsäure, Bromwasserstoffsäure, Toluolsulfonsäure, Methansulfonsäure und saure Ionenaustauscherharze.

Die Reaktion wird vorzugsweise in Gegenwart wassermischbarer organischer Verdünnungsmittel durchgeführt.

Als solche seien genannt:
niedere aliphatische Carbonsäuren wie Ameisensäure oder Essigsäure, Dioxan, Ethylenglykoldimethylether, Diethylenglykoldiethylether, Dimethylformamid, Dimethylsulfoxid, Sulfolan und N-Methyl-pyrrolidon.

Es wird bei Temperaturen zwischen etwa 50 °C und 180 °C, bevorzugt zwischen 70 °C und 160 °C gearbeitet.

Die Aufarbeitung erfolgt nach üblichen Methoden (vgl. Beispiele).

Setzt man bei Verfahren 1c) als Verbindung der Formel (II) 5,6-Dichlornikotinsäurechlorid und als Verbindung der Formel (V) Methylmagnesiumchlorid ein, läßt sich das Verfahren durch das folgende Reaktionsschema darstellen:

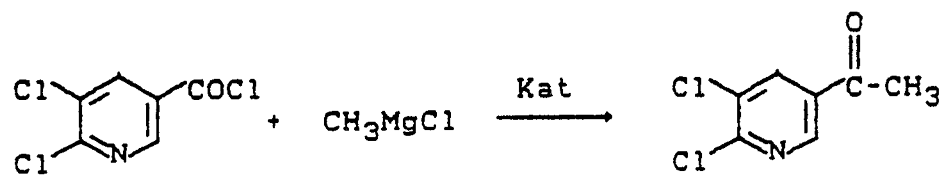


Die Verbindungen der Formel (V) sind bekannt. Im einzelnen seien genannt:
Methylmagnesiumchlorid, Methylmagnesiumbromid.

Die Reaktion wird durchgeführt, indem man die Nikotinsäurehalogenide und Methylmagnesiumhalogenid in Gegenwart eines Katalysators in einem Verdünnungsmittel umsetzt.

Als Katalysatoren seien genannt:
Eisen(II)chlorid, Tris-(2,4-pentandionato)-eisen.

Als Verdünnungsmittel kommen praktisch alle inerten organischen Lösungsmittel in Frage.
Hierzu gehören insbesondere aliphatische und aromatische Kohlenwasserstoffe wie Pentan, Hexan, Heptan, Cyclohexan, Petrolether, Benzin, Ligroin, Benzol, Toluol, ferner Ether wie Diethyl- und Dibutylether, Glykoldimethylether und Diglykoldimethylether, Tetrahydrofuran und Dioxan.

Pro Mol des Nikotinsäurehalogenids der Formel (II) wer den 0,8 - 1 Mol Methylmagnesiumhalogenid der Formel (V) und 1 - 10 Mol-% Katalysator eingesetzt.

Es wird bei Temperaturen von -100 °C bis +50 °C bevorzugt -80 °C bis 20 °C gearbeitet.

Es wird wie bei Grignard-Reaktionen üblich aufgearbeitet.

Verfahren 1d) läßt sich durch folgendes Reaktionsschema darstellen:

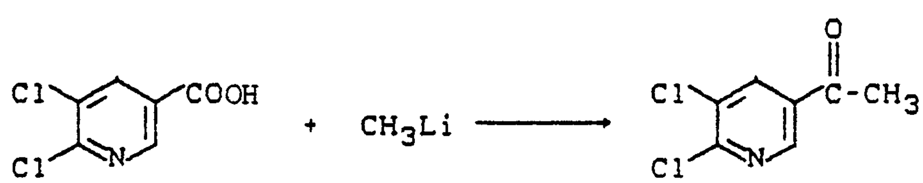

5,6-Dichlornikotinsäure ist bekannt (H. Meyer und R. Graf, Ber. Dt. Chem. Ges. 61, (1928), 2022).
Methyllithium ist bekannt.

Die Reaktion wird durchgeführt, indem man die Nikotinsäure mit Methyllithium in einem Verdünnungsmittel umsetzt.

Als Verdünnungsmittel kommen praktisch alle inerten organische Lösungsmittel in Frage.

Hierzu gehören insbesondere aliphatische und aromatische Kohlenwasserstoffe wie Pentan, Hexan, Heptan, Cyclohexan, Petrolether, Benzin, Ligroin, Benzol, Toluol, ferner Ether wie Diethyl- und Dibutylether, Glykoldimethyl ether und Diglykoldimethylether, Tetrahydrofuran und Dioxan.

Pro Mol Nikotinsäure der Formel (VI) werden 2 - 4 Mol Methyllithium eingesetzt.

Es wird bei Temperaturen von -100 °C bis +50 °C, bevorzugt von -90 °C bis 0 C gearbeitet.

Es wird wie bei Umsetzungen mit Lithium-organischen Verbindungen üblich aufgearbeitet.

Die neuen Verbindungen der Formel (IV) werden nach der in Verfahren 1a) beschriebenen Umsetzung von Nikotinsäurehalogeniden der Formel (II) mit Malonestern der Formel (III) hergestellt.

Das nach den erfindungsgemäßen Verfahren 1a-d) erhältliche 2,3-Dichlor-5-acetylpyridin der Formel (I) wird zur Herstellung von Pyridylethanolaminderivaten verwendet.

Dazu wird das Dichloracetylpyridin mit Ammoniak in Amino-chlor-acetyl-pyridin überführt, das mit elementarem Halogen oder mit Kupferhalogeniden umgesetzt wird. Das so erhaltene Halogenmethylpyridylketon wird mit Aminen umgesetzt und anschließend reduziert. Diese Reaktion läßt sich durch folgendes Formelschema illustrieren:

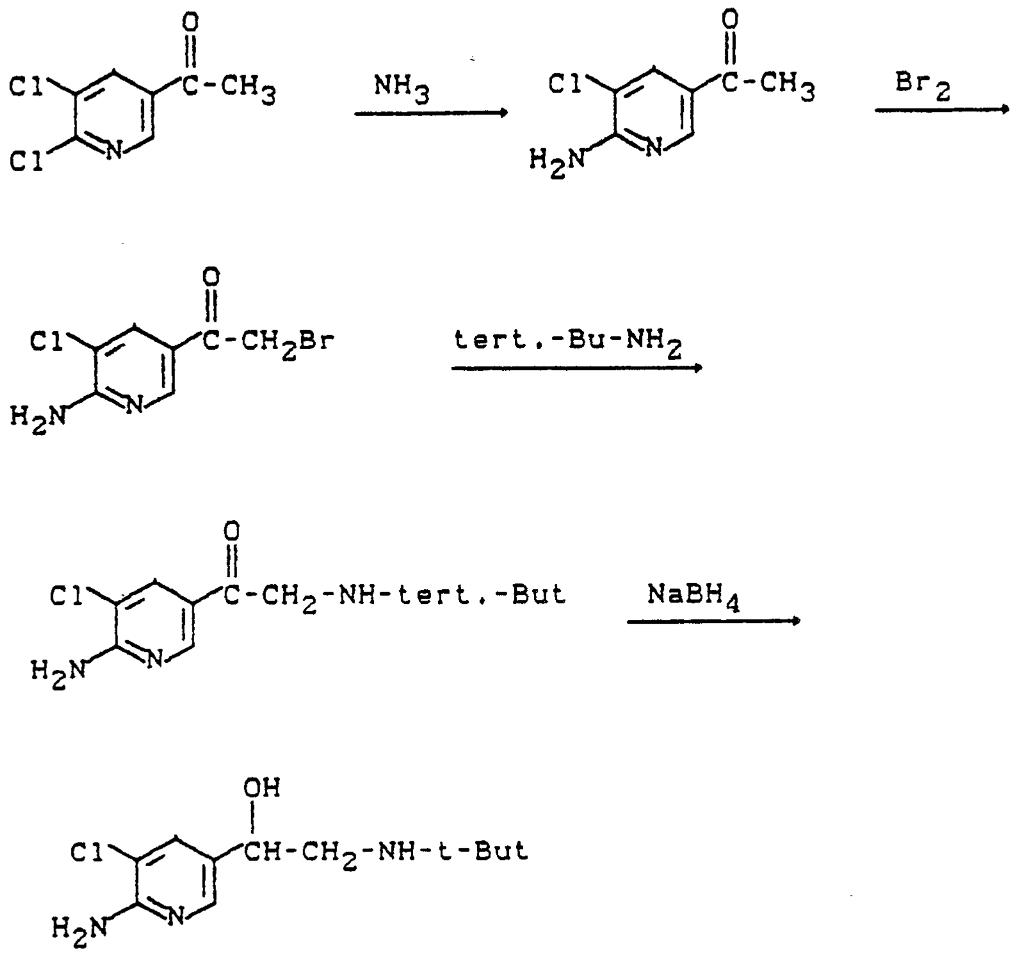


Das 2,3-Dichlor-5-acetyl-pyridin der Formel (I) kann auch in einem sogenannten "Eintopf-Verfahren" aus einem Dichlornicotinoylmalonsäureester der Formel (IV) erzeugt und ohne Zwischenisolierung zu 2-Amino-3-chlor-5-acetylpyridin (oben) weiter umgesetzt werden; z. B.:

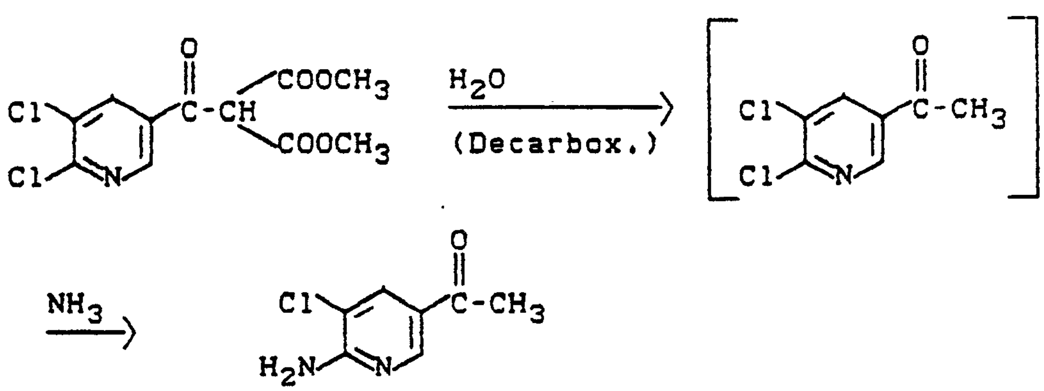

Die Halogenmethylketone können auch zunächst reduziert und dann mit Amin umgesetzt werden.

Die Durchführung der Folgereaktionen ist in einer nicht vorveröffentlichten Anmeldung der Anmelderin (Deutsche Patentanmeldung P 3 615 293.5) beschrieben.

## Herstellungsbeispiele

Beispiele für Verfahren 1a (1. Stufe) und 2

### Beispiel 1

Zu 10 ml trockenem Acetonitril werden bei 0°C 1,43 g (15 mmol) wasserfreies $MgCl_2$ gegeben. Nach Abklingen der exothermen Reaktion werden 1,7 ml (15 mmol) Malonsäuredimethylester und anschließend 4,2 ml (30 mmol) Triethylamin zugegeben, 15 Minuten bei 0°C gerührt. Zu der Mischung werden dann 2,63 g (12,5 mmol) 5,6-Dichlornikotinsäurechlorid (hergestellt nach H. Meyer und R. Graf, Ber. dt. Chem. Ges. 61 (1928) 2202), gelöst in 3 ml Dichlormethan zugetropft. Es wird noch eine Stunde bei 0°C, dann zwei Stunden bei Raumtemperatur gerührt. Zur Aufarbeitung wird auf 50 ml 1N Salzsäure gegeben, mit Dichlormethan extrahiert und der Extrakt mit gesättigter NaCl-Lösung gewaschen. Nach Trocknung mit $Na_2SO_4$ wird eingedampft.

Rückstand: 3,68 g (96 % der Theorie)

Schmelzpunkt: 99 °C

### Beispiel 2

5,6-Dichlor-nikotinoyl-malonsäurediethylester wird analog in 90 %iger Reinheit als Öl erhalten.

Beispiel für die Verwendung von NaOH/MgO als Base

### Beispiel 3

8,4 ml (55 mmol) Malonsäurediethylester werden in 25 ml trockenem Dimethylformamid bei 0 °C vorgelegt und 6,6 g (55 mmol) einer feingepulverten Mischung aus einem Gewichtsteil MgO und zwei Gewichtsteilen NaOH eingetragen. Es wird noch 15 Minuten bei 0 °C gerührt, dann 5,3 g (25 mmol) 5,6-Dichlornikotinsäurechlorid, gelöst in 5 ml trockenem Dichlormethan bei 0 °C eingetropft. Dann wird eine Stunde bei 0 °C und anschließend noch zwei Stunden bei Raumtemperatur gerührt. Zur Aufarbeitung wird das Ganze auf 150 ml 2N HCl-Lösung gegossen, mit Dichlormethan extrahiert. Die vereinigten Extrakte werden mehrmals mit gesättigten NaCl-Lösung gewaschen, mit $Na_2SO_4$ getrocknet und eingedampft.

Rückstand: 7,0 g gelbes Öl

Gehalt an 5,6-Dichlornikotinoylmalonsäurediethylester: 67 %.

Beispiel für die Verwendung von K-tert.-Butylat als Base

### Beispiel 4

2,86 g (30 mmol) wasserfreies $MgCl_2$ werden in 10 ml trockenem Acetonitril vorgelegt. Dann werden bei 0 °C 4,6 ml (30 mmol) Malonsäurediethylester zugegeben und anschließend 6,75 g (60 mmol) K-tert.-Butylat portions weise bei 0 °C eingetragen. Zu dieser Mischung wird bei 0 °C eine Lösung von 6,25 g (25 mmol) 5,6-Dichlor-nikotinsäurechlorid, gelöst in 10 ml trockenem Dichlormethan zugetropft. Es wird zuerst eine Stunde bei 0 °C, dann zwei Stunden bei Raumtemperatur nachgerührt, anschließend auf 150 ml $^2$N HCl-Lösung gegossen, mehrmals mit Dichlormethan extrahiert, mit $Na_2$-$SO_4$ getrocknet und eingedampft.

Rückstand: 9,1 g gelbes Öl.

Gehalt an 5,6-Dichlornikotinoylmalonsäurediethylester: 42 %

Beispiel für Verfahren 1a (2. Stufe) und 1b

Beispiel 5

5-Acetyl-2,3-dichlorpyridin

30,4 g (Gehalt 90 %, 82 mmol) 5,6-Dichlor-nikotinoylmalonsäurediethylester werden zu einer Mischung von 123 ml Eisessig, 77 ml Wasser und 4,45 g (45 mmol) 96 %iger $H_2SO_4$ gegeben und das Ganze auf 100 °C erhitzt. Nach 120 Minuten sind etwa 90 % des Malonesters umgesetzt. Es wird abgekühlt, auf 500 ml Wasser gegossen, mit Dichlormethan extrahiert und der Extrakt zweimal mit 1N Natronlauge gewaschen. Nach Trocknung mit $Na_2SO_4$ wird eingedampft.
Ausbeute: 9,3 g (60 % der Theorie)
Schmelzpunkt: 80 °C

Beispiel für die Verwendung von Dioxan

Beispiel 6

3,04 g (7,3 mmol) 5,6-Dichlornikotinoylmalonsäurediethylester (Gehalt 80 %) werden zu einer Mischung von 17 ml Dioxan, 7,5 ml $H_2O$ und 970 mg (9,1 mmol) $H_2SO_4$ gegeben und das Ganze 5 Stunden unter Rückfluß erhitzt. Zur Aufarbeitung wird mit NaOH neutralisiert, eingedampft und der Rückstand in 100 ml 10 %igen NaOH aufgenommen. Es wird mit Dichlormethan extrahiert, mit 10 %igen $NaH_2PO_4$-Lösung gewaschen und nach Trocknung mit $Na_2SO_4$ eingedampft.
Rückstand: 1,1 g (79,5 % der Theorie)
Schmelzpunkt: 80 °C

Beispiel für die Verwendung von Dimethylsulfoxid

Beispiel 7

Eine Mischung aus 15,3 g (0,048 Mol) 5,6-Dichlornicotinoylmalonsäuredimethylester (96,5 %ig), 1,8 g (0,10 Mol) Wasser und 50 ml Dimethylsulfoxid wird 3 Stunden bei 150 °C Badtemperatur gerührt und dann in 125 ml Wasser gegossen. Die organische Phase wird abgetrennt und mit 100 ml Methylenchlorid verdünnt. Die wäßrige Phase wird mit Methylenchlorid geschüttelt. Die vereinigten Methylenchlorid-Phasen werden mit Natriumsulfat getrocknet und filtriert. Vom Filtrat wird das Lösungsmittel im Wasserstrahlvakuum sorgfältig abdestilliert.

Man erhält 10,9 g Rohprodukt, das nach GC-MS-Analyse 83 % 2,3-Dichlor-5-acetylpyridin (99 % der Theorie) und 12 % Dimethylsulfoxid sowie 3 % 2-Chlor-5-acetylpyridin enthält.

Beispiel für Verfahren 1c

Beispiel 8

Zu einer Lösung von 3,7 g (17,6 mmol) 5,6-Dichlor-nikotinsäurechlorid in 36 ml trockenem Tetrahydrofuran werden 310 mg (0,88 mmol) Tris-(2,4-pentandionato)-eisen zugegeben und anschließend bei -20 °C 5 ml (15 mmol) einer 3 molaren Lösung von Methylmagnesiumchlorid in Tetrahydrofuran in einem Zeitraum von 30 Minuten zugetropft. Es wird noch 20 Minuten bei -20 °C nachgerührt, dann auf 300 ml 1 N HCl

gegossen und mit Dichlormethan zuschöpfend extrahiert. Die vereinigten Extrakte werden nacheinander mit 2 N NaOH, dann mit 5 %iger $NaH_2PO_4$-Lösung gewaschen, mit $Na_2SO_4$ getrocknet und eingedampft.
Rückstand: 1,75 g (61 % der Theorie, bezogen auf eingesetztes Methylmagnesiumchlorid)
Schmelzpunkt: 78 °C.

Beispiel für Verfahren 1d

Beispiel 9

Zur Lösung von 9,6 g (50 mmol) 5,6-Dichlornikotinsäure in 250 ml abs. Ether werden bei 0 °C 62 ml einer 1,6 M Lösung (100 mmol) von Methyllithium in Ether gegeben. Nach beendeter Zugabe wird 30 min bei 0 °C nachgerührt, dann unter Eiskühlung mit 200 ml Wasser versetzt. Man trennt die Phasen und extrahiert die Wasserphase noch einmal mit 50 ml Ether. Die vereinigten organischen Phasen werden über Natriumsulfat getrocknet und eingedampft.

Man erhält 9 g (95 % der Theorie) 2,3-Dichlor-5-acetylpyridin, Schmelzpunkt 80 °C.

Beispiele für die weitere Umsetzung des 5,6-Dichloracetylpyridins zu substituierten Pyridylethanolaminen

Beispiel a (Aminolyse)

2-Amino-3-chlor-5-acetylpyridin

Variante α

1,9 g (10 mmol) 2,3-Dichlor-5-acetylpyridin werden in einer Mischung von 80 ml Tetrahydrofuran und 20 ml konzentriertem wäßrigem Ammoniak im Autoklaven 8 Stunden bei 170 °C erhitzt. Nach Abdampfen des Tetrahydrofurans wird mit Wasser verdünnt, auf pH 5 gestellt und mit Essigester extrahiert.
Ausbeute: 1,65 g (97 %), Fp.: 188 °C

Variante β

Eine Mischung aus 72,3 g (0,38 Mol) 2,3-Dichlor-5- acetyl-pyridin und 380 ml Dimethylsulfoxid wird auf 70 °C erwärmt und bei dieser Temperatur werden 760 ml einer konzentrierten wäßrigen Ammoniaklösung tropfenweise dazu gegeben. Das Reaktionsgemisch wird 19 Stunden bei 100 °C gerührt und auf 15 °C abgekühlt. Das hierbei kristallin angefallene Produkt wird durch Absaugen isoliert.
Ausbeute: 53,1 g (82 % der Theorie), Fp. 188 °C.

Variante γ

Eine Mischung aus 19,0 g (0,10 Mol) 2,3-Dichlor-5-acetyl-pyridin, 300 ml Isopropanol und 110 ml einer konzentrierten wäßrigen Ammoniaklösung wird 5 Stunden bei 170 °C im Autoklaven gerührt. Anschließend wird eingeengt, der Rückstand 30 Minuten mit 400 ml Wasser verrührt und das kristallin angefallene Produkt durch Absaugen isoliert.
Ausbeute: 16,5 g (97 % der Theorie), Fp. 188 °C.

Variante δ

Eine Mischung aus 15,3 g (0,0483 Mol) 2,3-Dichlor-5-nicotinoylmalonsäure-dimethylester, 50 ml Dimethylsulfoxid und 1,8 g Wasser wird 3 Stunden bei 150 °C gerührt. Nach Abkühlen auf 90 °C werden 100 ml einer konzentrierten wäßrigen Ammoniaklösung tropfenweise dazu gegeben. Das Reaktionsgemisch wird

18 Stunden bei 100 °C gerührt und dann auf 15 °C abgekühlt. Dass hierbei kri stallin angefallene Produkt wird durch Absaugen isoliert.
Aubeute: 6,6 g (80 % der Theorie).

Beispiel b (Halogenierung)

2-Amino-3-chlor-5-pyridyl-(brommethyl)keton

Zu einer Lösung von 17,05 g (0,1 mol) 2-Amino-3-chlor-5-acetylpyridin in einer Mischung von 19,3 g Bromwasserstoff (47 %ige wäßrige Lösung; 0,11 mol) und 500 ml Eisessig werden 16 g (0,1 mol) Brom getropft. Man rührt zwei Stunden nach, stellt die Mischung auf pH 8 und extrahiert mit Essigester. Nach Trocknen und Eindampfen erhält man 18,5 g (74 %) der Titelverbindung, Fp. 134 °C

Beispiel c (Umsetzung des Pyridylhalogenalkylketons mit Aminen)

2-Amino-3-chlor-5-pyridyl-(isopropylaminomethyl)keton

In eine Lösung von 11,8 g (0,2 mol) Isopropylamin in 150 ml Methanol werden bei 0 °C portionsweise 9,98 g (0,04 mol) der unter Beispiel b hergestellten Verbindung eingetragen. Man läßt auf Raumtemperatur kommen, rührt 2 Stunden nach und dampft ein. Der Rückstand wird in Puffer pH 5 aufgenommen und mit Ether gewaschen. Die wäßrige Phase wird auf pH 9 gestellt und mit Essigester extrahiert. Nach Trocknen und Eindampfen werden 6,8 g (75 %) der Titelverbindung als amorphes Pulver erhalten.

Beispiel d (Reduktion des Pyridylaminoalkylketons)

1-(2-Amino-3-chlor-5-pyridyl)-2-isopropylaminoethanol

Zur Lösung von 2,28 g (10 mmol) der nach Beispiel c hergestellten Verbindung in 50 ml Methanol werden bei 0 °C portionsweise 0,38 g (10 mmol) Natriumborhydrid gegeben. Man läßt auf Raumtemperatur kommen, stellt mit verdünnter Salzsäure auf pH 1 und dampft ein. Der Rückstand wird in Wasser aufgenommen und mit Ether gewaschen. Dann wird auf pH 10 gestellt und mit Essigester extrahiert. Nach Trocknen und Eindampfen erhält man 2,1 g (92 %) der Titelverbindung, Fp. 146 °C

## Ansprüche

1. Verfahren zur Herstellung von 2,3-Dichlor-5-acetylpyridin der Formel (I)

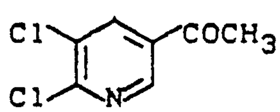

(I)

a) dadurch gekennzeichnet, daß man 5,6-Dichlornikotinsäurehalogenide der Formel (II)

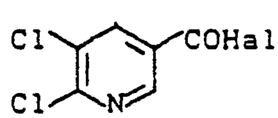

(II)

in welcher
Hal für Halogen steht,

mit Malonsäureestern der Formel (III)

$CH_2(COOR^1)_2$ (III)

in welcher

$R^1$ für $C_{1-3}$-Alkyl steht,

in Gegenwart von Magnesiumverbindungen und Basen umsetzt und anschließend schonend in Gegenwart wäßriger Säuren decarboxyliert, oder

b) dadurch gekennzeichnet, daß man Verbindungen der Formel (IV)

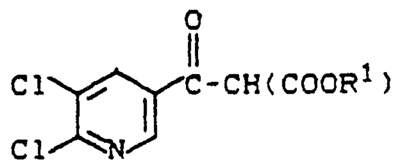

(IV)

in welcher

$R^1$ für $C_{1-3}$-Alkyl steht

in Gegenwart von Wasser, gegebenenfalls in Gegenwart eines organischen Lösungsmittels und gegebenenfalls in Gegenwart einer Protonensäure, bei Temperaturen zwischen 50 °C und 180 °C decarboxyliert, oder

c) dadurch gekennzeichnet, daß man 5,6-Dichlornikotinsäurehalogenide der Formel (II)

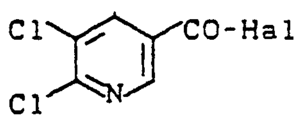

(II)

mit Methylmagnesiumhalogenid der Formel (V)

$H_3C$ MgHal (V)

in welcher

Hal für Halogen steht,

in Gegenwart von Katalysatoren umsetzt, oder

d) dadurch gekennzeichnet, daß man 5,6-Dichlornikotinsäure der Formel (VI)

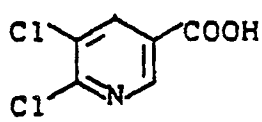

(VI)

mit Methyllithium umsetzt.

2. Verbindungen der Formel (IV)

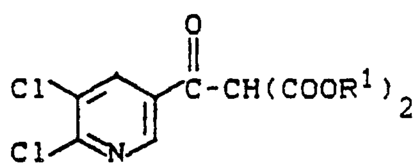

(IV)

in welcher

$R^1$ für $C_{1-3}$-Alkyl steht.

3. Verfahren zur Herstellung der Verbindungen der Formel (IV) gemäß 2 (oben), dadurch gekennzeichnet, daß man Verbindungen der Formel (II)

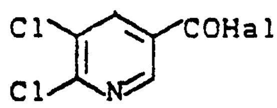

(II)

in welcher
Hal für Halogen steht,
mit Malonsäureestern der Formel (III)

$CH_2(COOR^1)_2$ (III)

in welcher
$R^1$ für $C_{1-3}$-Alkyl steht,
in Gegenwart von Magnesiumsalzen und Basen umsetzt.